# EUROPEAN PATENT SPECIFICATION

(11) **EP 2 931 271 B1**
(45) Date of publication and mention of the grant of the patent: **23.08.2017**
(21) Application number: 13814047.0
(22) Date of filing: 22.11.2013
(51) Int. Cl.: A61K 31/375, A61K 38/39, A61K 8/65, A61K 8/67, A61P 17/00, A61P 19/10, A61P 19/02, A61K 8/64, A61Q 19/08, A61K 9/00

(54) **COMPOSITIONS CONTAINING COLLAGEN, ELASTIN HYDROLYSATES AND VITAMIN C PROMOTING THE DEPOSIT OF EXTRACELLULAR MATRIX**
ZUSAMMENSETZUNGEN MIT KOLLAGEN- UND ELASTIN-HYDROLYSATEN UND VITAMIN C ZUR FÖRDERUNG DER ABLAGERUNG EINER EXTRAZELLULÄREN MATRIX
COMPOSITIONS CONTENANT DES HYDROLYSATS DE COLLAGÈNE ET D'ÉLASTINE ET DE LA VITAMINE C, QUI FAVORISENT LE DÉPÔT D'UNE MATRICE EXTRACELLULAIRE

(30) Priority: 14.12.2012 IT MI20122145
(43) Date of publication of application: 21.10.2015
(73) Proprietor: Velleja Research SRL, 29010 Pontenure (PC) (IT)
(72) Inventor: DI PIERRO, Francesco, I-29010 Pontenure (PC) (IT)
(74) Representative: Bianchetti Bracco Minoja S.r.l.
(86) International application number: PCT/EP2013/074458
(87) International publication number: WO 2014/090546

(56) References cited:
- EP-A1- 2 468 307
- WO-A1-93/13757
- US-A- 5 578 307
- US-A1- 2005 158 396

## Description

The present invention relates to compositions containing collagen hydrolysate, elastin hydrolysate and vitamin C, which promote the deposit of extracellular matrix.

### Prior art

Collagen is the main protein of connective tissue. It is the most abundant protein in mammals (about 25% of the total protein mass), and represents about 6% of human body weight. There are numerous types of collagen properly so called, and various proteins which have a polypeptide structure very similar to collagen. 28 types of collagen have been described in the literature to date. The four best known and most widely studied types are type I, which represents 90% of total collagen and is a component of the main connective tissues, such as skin, tendon, bone and cornea; type II, which is abundant in cartilage, intervertebral discs and vitreous humour; type III, which is very important in the cardiovascular system, is also the collagen of granulation tissue, and is produced rapidly and in large quantities before the stronger type I collagen, by which it is subsequently replaced; and type IV, which forms the basal membrane.

The collagen obtained by extraction from bovine connective tissue subjected to enzymatic digestion with microbial collagenase generates collagen fragments (the mixture of which is called "collagen hydrolysate") with molecular weights ranging from 0.5 to 25 KDa. Said fragments are orally bioavailable for values exceeding 90% of the dose administered, and exhibit major tropism for tissues (dermis and connective tissue, joint cartilage, synovial fluid and bone) rich in fibroblasts, chondrocytes, osteoblasts and synovial fluid cells. The oral use of said hydrolysate is also described as providing advantages in the clinical field (faster healing of skin wounds, reduced joint damage in patients suffering from osteoarthritis, reduced pain in athletes with joint and/or ligament traumatisms, and slowing of osteoporotic degeneration in elderly patients with senile osteoporosis).

These clinical benefits seem to be associated with receptor-mediated molecular recognition of said hydrolysed collagen fragments by the cell species described above. *In vitro* tests demonstrate that incubating a culture of bovine chondrocytes with bovine collagen hydrolysate increases the efficiency of deposit of cartilage tissue without generating proliferation aspects. This phenomenon does not seem to be related in any way to the type of collagen used, only to the target cells used. The use of type I collagen hydrolysate promotes the deposit of type I or II collagen, depending on the cell culture used. Equally, the use of type II collagen hydrolysate produces deposits of type I and type II collagen simply due to different use of the various cell types (chondrocytes always produce type II, and fibroblasts always produce type I).

EP 2 468 307 discloses a composition comprising elastin hydrolysates and collagen matrices cross-linked by means of epoxy-functional cross-linking agents, for use as a cosmetic mask, as subcutaneous implant, as dermis replacements, as scaffold for cell populations and as wound dressings.

US 5 578 307 discloses shaped articles containing plant extracts dispersed in a matrix composed of collagen, gelatin, plant proteins or hydrolysate thereof.

### Description of the invention

It has now been found that the deposit of extracellular matrix by fibroblasts, chondrocytes, osteoblasts and synovial fluid cells is boosted synergically by the presence of collagen hydrolysate, elastin hydrolysate and vitamin C.

The object of the invention is therefore combinations of collagen hydrolysate, elastin hydrolysate and vitamin C, for use to optimise the processes of deposit of extracellular matrix starting with the activation of fibroblasts, chondrocytes, osteoblasts and synovial fluid cells.

Said processes are advantageous in the prevention and treatment of:
1) *Striae rubrae* in adolescents caused by weight variations
2) *Striae gravidarum*
3) Systemic Lupus erythematosus
4) Panarterite nodosa
5) Dermatomyositis
6) Lichen sclerosus et atrophicus of the mucosa
7) Post-operative hypertrophic or keloid scars or sequelae of burns
8) Sequelae of plastic surgery
9) Skin aging
10) Panniculopathies
11) Tendinopathies and myopathies
12) Traumatisms (kneecap, ligaments, joint capsules, fractures)
13) Osteoarthritis (knee, hip, lumbosacral)
14) Senile osteoporosis.

In particular, the combination of collagen hydrolysate and elastin hydrolysate in the ratio of 10:1 has proved effective, regardless of the dose of vitamin C added.

The compositions according to the invention can be administered topically or orally. Formulations suitable for topical administration comprise creams, gels and ointments, water-in-oil and oil-in-water emulsions, and sprays for dermatological use, containing collagen in concentrations ranging from 0.1 to 2%, preferably from 1 to 2%; elastin hydrolysate from 0.1 to 2%, preferably from 0.1 to 0.5%; and vitamin C from 0.1 to 2%, preferably from 0.5 to 1%.

Formulations suitable for oral use are tablets, capsules or powders containing between 100 mg and 10 g, preferably 5 g, of collagen hydrolysate; between 10 mg and 1 g, preferably 500 mg, of elastin hydrolysate; and between 1 and 1000 mg, preferably between 5 and 200 mg, of vitamin C.

Hydrolysed elastin is obtained by extracting elastin from fish skin, denaturing it with heat and then subjecting it to digestion with bacterial elastase. Water-soluble peptides weighing about 1400 Da are obtained, which have the function of elastase inhibitors. However, they may also have the function of cell activators, as observed with hydrolysed collagen.

To investigate their synergic potential, human fibroblast cultures were prepared with RPMI and FCS (5%) at 37°C, with 5% oxygen, 5% CO₂ and 90% molecular nitrogen. The culture was deemed suitable at t=0 with a cell content of 350,000 cells/cm². Under said conditions, the new collagen production capacity was tested using type I collagen hydrolysate (1 mg/ml) (test A), elastin hydrolysate (1 mg/ml) (test B), vitamin C (1 mg/ml) (test C), or A+B, A+C, B+C, A+B+C. Test D indicates the culture in the absence of stimuli.

Each test was conducted in triplicate. The analysis was performed after 7 days' culture. The results clearly demonstrate a considerable additive effect in test A+B+C, as shown in Table 1.

| **Table 1. Production of type I collagen in human fibroblast culture stimulated for 24 hours** | | |
|---|---|---|
| *Test* | *µg of collagen*/*10⁶ cells* | *p (vs test D)* |
| A | 1.8±0.5 | < 0.05 |
| B | 1.2±0.3 | N.S |
| C | 0.6±0.4 | N.S. |
| A+B | 4.2±1.1 | < 0.01 |
| A+C | 1.9±0.8 | < 0.05 |
| B+C | 1.4±0.6 | N.S. |
| A+B+C | 6.7±2.3 | <0.01 |
| D | 0.8±0.3 | |

The use of different doses of A, B and C was then investigated under the same experimental conditions as first described. In particular, the doses of the 3 ingredients studied were reduced by 90%. As shown in Table 2, collagen hydrolysate (0.1 mg/ml) combined with elastin hydrolysate (0.1 mg/ml) and vitamin C (0.1 mg/ml) exhibits an excellent ability to stimulate new collagen production.

Moreover, in an attempt to find ratios between the doses of collagen and elastin hydrolysate which are even more conducive to new collagen synthesis (as molecular competition phenomena cannot be ruled out), the action of the 2 hydrolysates was tested at the ratios of 1:1, 10:1, 100:1, 1:10 and 1:100. As shown in Table 3, the use of the ratio 10:1 in favour of collagen hydrolysate produced the best effect.

**Table 2. Production of type I collagen in human fibroblast culture stimulated for 24 hours with doses 90% lower than those used in the test shown in Table 1.**

| *Test* | *µg of collagen*/*10⁶ cells* | *p (vs test D)* |
|---|---|---|
| A | 0.8±0.3 | N.S. |
| B | 0.6±0.1 | N.S |
| C | 0.7±0.2 | N.S. |
| A+B+C | 5.2±1.1 | < 0.01 |
| D | 0.7±0.4 | |

**Table 3. Production of type I collagen in human fibroblast culture stimulated for 24 hours with different dose ratios between collagen hydrolysate and elastin hydrolysate. In terms of dose, 1 indicates 0.1 mg/ml; 10 indicates 1 mg/ml and 100 indicates 10 mg/ml.**

| *Test* | *µg of collagen*/*10⁶ cells* | *p (vs test D)* |
|---|---|---|
| 1:1 | 4.9±1.0 | < 0.01 |
| 10:1 | 5.8±1.1 | < 0.01 |
| 100:1 | 1.7±2.2 | < 0.01 |
| 1:10 | 3.2±0.9 | < 0.01 |
| 1:100 | 1.4±0.7 | < 0.05 |
| D | 0.6±0.2 | |

The demonstration of the usefulness of vitamin C, added in the test wherein collagen and elastin hydrolysate were tested in the ratio of 10:1, is particularly important. In said test, the addition of doses of vitamin C ranging from 0.01 to 0.5 produced a further 25% increase in collagen production, regardless of the dose of vitamin C used (data not shown).

Some examples of formulations according to the invention are set out below.

### Example 1

Cream for topical use:
- collagen hydrolysate: 1%
- elastin hydrolysate: 0.1%
- vitamin C: 0.5%

### Example 2 (not according to the present invention)

Spray for dermatological use:
- collagen hydrolysate: 0.5%
- elastin hydrolysate: 0.05%
- vitamin C: 0.25%

### Example 3 (not according to the present invention)

Powder for oral sachets (mg/dose)
- collagen hydrolysate: 5000 mg
- elastin hydrolysate: 500 mg
- vitamin C: 12 mg.

## Claims

1. Compositions containing collagen hydrolysate in concentrations ranging from 0.1 to 2%, preferably from 1 to 2%; elastin hydrolysate from 0.1 to 2%, preferably from 0.1 to 0.5%; and vitamin C from 0.1 to 2%, preferably from 0.5 to 1%.

2. Compositions according to claim 1 in the form of topical preparations in the form of cream, gel, ointment, water-in-oil or oil-in-water emulsions or sprays for dermatological use.

3. The compositions of claims 1-2 for use in the treatment and prevention of: *Striae rubrae* in adolescents caused by weight variations, *Striae gravidarum,* systemic lupus erythematosus, panarterite nodosa, dermatomyositis, lichen sclerosus et atrophicus of the mucosa, post-operative hypertrophic or keloid scars or sequelae of burns, sequelae of plastic surgery, skin aging, panniculopathies, tendinopathies and myopathies, traumatisms, osteoarthritis and senile osteoporosis.

## Patentansprüche

1. Zusammensetzungen, umfassend Kollagenhydrolysat in Konzentrationen, die von 0,1 bis 2% reichen, vorzugsweise von 1 bis 2%; Elastinhydrolysat von 0,1 bis 2%, vorzugsweise von 0,1 bis 0,5%; und Vitamin C von 0,1 bis 2%, vorzugsweise von 0,5 bis 1%.

2. Zusammensetzungen gemäß Anspruch 1 in der Form topischer Zubereitungen in Form von Creme, Gel, Salbe, Wasser-in-Öl- oder Öl-in-Wasser-Emulsionen oder Sprays für die dermatologische Verwendung.

3. Zusammensetzungen gemäß Ansprüchen 1-2 zur Verwendung in der Behandlung und Vorbeugung von *Striae rubrae* in Heranwachsenden, verursacht durch Gewichtsveränderungen, *Striae gravidarum,* systemischen Lupus erythematodes, Panarterite nodosa, Dermatomyositis, Lichen sclerosus et atrophicus der Mucosa, post-operative hypertrophe oder keloide Narben oder Folgekrankheiten von Verbrennungen, Folgekrankheiten plastischer Chirurgie, Hautalterung, Cellulite, Tendopathien und Myophatien, Traumata, Osteoarthritis und senile Osteoporose.

## Revendications

1. Compositions contenant un hydrolysat de collagène dans des concentrations allant de 0,1 à 2 %, de préférence de 1 à 2 % ; un hydrolysat d'élastine de 0,1 à 2 %, de préférence de 0,1 à 0,5 % ; et de la vitamine C de 0,1 à 2 %, de préférence de 0,5 à 1 %.

2. Compositions selon la revendication 1 sous la forme de préparations topiques sous la forme de crème, de gel, de pommade, d'émulsions eau-dans-huile ou huile-dans-eau ou de pulvérisations à usage dermatologique.

3. Compositions selon les revendications 1 à 2, pour une utilisation dans le traitement et la prévention de :
*Striae rubrae* chez les adolescents causée par des variations de poids, *Striae gravidarum,* du lupus érythémateux systémique, de la polyartérite noueuse, de la dermatomyosite, du lichen scléreux et atrophique des muqueuses, des cicatrices hypertrophiques ou chéloïdes post-opératoires ou des séquelles de brûlures, des séquelles de chirurgie plastique, du vieillissement cutané, des panniculopathies, des tendinopathies et des myopathies, des traumatismes, de l'ostéoarthrite et l'ostéoporose sénile.
